(19) Europäisches Patentamt
European Patent Office
Office européen des brevets

(11) EP 3 269 423 A1

(12) # EUROPÄISCHE PATENTANMELDUNG

(43) Veröffentlichungstag:
**17.01.2018 Patentblatt 2018/03**

(51) Int Cl.:
***A61N 1/375*** (2006.01) ***A61N 1/372*** (2006.01)

(21) Anmeldenummer: **16179171.0**

(22) Anmeldetag: **13.07.2016**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(71) Anmelder: **BIOTRONIK SE & Co. KG**
**12359 Berlin (DE)**

(72) Erfinder:
- **Moß, Christian**
**10589 Berlin (DE)**
- **Gothe, Christian**
**12437 Berlin (DE)**
- **Finnberg, Thomas**
**8037 Zürich (CH)**

(74) Vertreter: **Randoll, Sören**
**Biotronik SE & Co. KG**
**Corporate Intellectual Property**
**Woermannkehre 1**
**12359 Berlin (DE)**

(54) **IMPLANTIERBARES MEDIZINISCHES GERÄT MIT ANTENNE FÜR DRAHTLOSE KOMMUNIKATION**

(57) Die Erfindung betrifft ein implantierbares medizinisches Gerät (10), das ein Hohlgehäuse (12) und ein Anschlussgehäuse (14) aufweist. Das Anschlussgehäuse (14) ist mit dem Hohlgehäuse (12) verbunden, insbesondere fest verbunden. Sowohl das Hohlgehäuse (12) als auch das Anschlussgehäuse (14) enthalten elektrische Komponenten, von denen die in dem Hohlgehäuse (12) enthaltenen elektrischen Komponenten wenigstens einen Empfänger (24) für drahtlose Kommunikation umfassen und die in dem Anschlussgehäuse (14) enthaltenen Komponenten wenigstens eine Antenne (20) umfassen die zum Empfangen elektromagnetischer Wellen mit einer Frequenz von mehr als 100 MHz konfiguriert ist und die elektrisch mit dem Empfänger (24) verbunden ist. Ein derartiger Empfänger (24) der mit einer derartigen Antenne verbunden ist, erlaubt beispielsweise einen Empfang von Daten in dem MICS-Frequenzband. Erfindungsgemäß umfassen die in dem Anschlussgehäuse (14) angeordneten elektrischen Komponenten außerdem einen Schwingkreis (22) der eine Spule (26) und eine Kapazität (28) aufweist und der induktiv mit der Antenne (20) gekoppelt ist. Der Schwingkreis (22) ist konfiguriert auf magnetische Wechselfelder in einem Frequenzbereich zwischen 5 kHz und 50 MHz anzusprechen und hat entsprechend vorzugsweise eine Resonanzfrequenz zwischen 5 kHz und 50 MHz. Der Schwingkreis (22) ist damit für eine Datenkommunikation mittels magnetischer Wechselfelder im Nahfeld des implantierbaren medizinischen Geräts (10) geeignet.

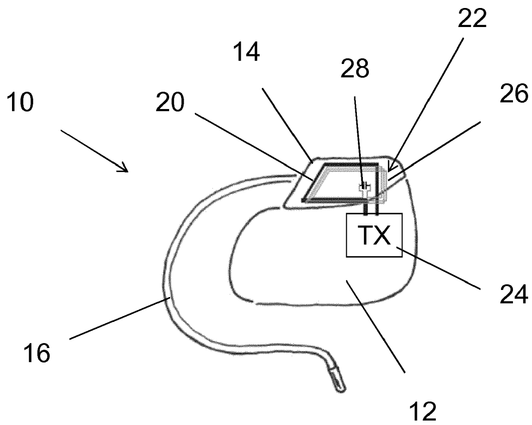


Fig. 1

EP 3 269 423 A1

## Beschreibung

Die Erfindung betrifft ein implantierbares medizinisches Gerät, beispielsweise ein implantierbares Therapiegerät, das ein Hohlgehäuse und ein Anschlussgehäuse aufweist, bei dem das Anschlussgehäuse mit dem Hohlgehäuse verbunden ist und wobei sowohl das Hohlgehäuse als auch das Anschlussgehäuse elektrische Komponenten enthalten. Die in dem Hohlgehäuse enthaltenen elektrischen Komponenten umfassen einen Empfänger für eine drahtlose Kommunikation und die in dem Anschlussgehäuse enthaltenen Komponenten umfassen eine Antenne, die elektrisch mit dem Empfänger in dem Hohlgehäuse verbunden ist.

Derartige implantierbare medizinische Geräte können solche Therapiegeräte wie beispielsweise Stimulationsgeräte sein. Solche Stimulationsgeräte sind beispielsweise Herzschrittmacher, Defibrillatoren oder auch Neurostimulatoren. Andere implantierbare aktive medizinische Geräte sind Monitorgeräte, um beispielsweise physiologische Parameter zu Erfassen und Aufzuzeichnen. In vielen Fällen weisen diese implantierbaren medizinischen Geräte ein Anschlussgehäuse auf, welches Anschlüsse zum Anschluss von elektrischen Leitungen aufweist, die beispielsweise Stimulationselektrodenleitungen sein können oder aber auch Sensorleitungen. Das Anschlussgehäuse ist häufig als Kunststoff (Guss-)teil ausgeführt, welches mit einem Hohlgehäuse verbunden ist, das weitere Komponenten des implantierbaren medizinischen Gerätes umfasst wie beispielsweise eine Batterie, Auswertungselektronik oder dergleichen. Typischerweise ist das Hohlgehäuse aus einem biokompatiblen Metall gefertigt. In dem Hohlgehäuse kann neben den bereits genannten Komponenten auch ein Telemetriesender und/oder -empfänger vorgesehen sein um uni- oder bidirektional drahtlos mit dem implantierbaren medizinischen Gerät kommunizieren zu können. Dies ist insbesondere in Zusammenhang mit der an sich bekannten Telemedizin interessant, bei der ein implantierbares medizinisches Gerät physiologische Parameter erfasst und speichert und telemetrisch an ein externes Gerät überträgt. Derartige Daten können beispielsweise ein Intrakardiales-Elektrokardiogramm oder dergleichen repräsentieren. Außerdem kann über eine derartige durch den Telemetriesender und -empfänger realisierte Telemetrie auch ein Übertragen von Daten zu dem implantierbaren medizinischen Gerät erfolgen. Der hierfür eingesetzte Sender und/oder Empfänger arbeitet typischerweise in einem Frequenzbereich zwischen 400 MHz und 460 MHz (MICS/MEDS bzw. MedRadio) und in weiteren für die Implantatskommunikation zugelassenen Funkbändern.

Neben einer Datenkommunikation im MICS-Band erlauben programmierbare implantierbare medizinische Geräte häufig auch eine Nahfeldkommunikation bei der beispielsweise ein externes Programmiergerät mit dem implantierbaren medizinischen Gerät über ein magnetisches Wechselfeld (H-Feld) kommuniziert. Diese Form der Datenkommunikation ist besonders energiegünstig.

Aufgabe der Erfindung ist es, ein implantierbares medizinisches Gerät zu schaffen, das eine effiziente drahtlose Kommunikation erlaubt.

Erfindungsgemäß wird diese Aufgabe durch ein implantierbares medizinisches Gerät gelöst, das ein Hohlgehäuse und ein Anschlussgehäuse aufweist. Das Anschlussgehäuse ist mit dem Hohlgehäuse verbunden, insbesondere fest verbunden. Sowohl das Hohlgehäuse als auch das Anschlussgehäuse enthalten elektrische Komponenten, von denen die in dem Hohlgehäuse enthaltenen elektrischen Komponenten wenigstens einen Empfänger für drahtlose Kommunikation umfassen und die in dem Anschlussgehäuse enthaltenen Komponenten wenigstens eine Antenne die zum Empfangen elektromagnetischer Wellen mit einer Frequenz von mehr als 100 MHz konfiguriert ist und die elektrisch mit dem Empfänger verbunden ist. Ein derartiger Empfänger der mit einer derartigen Antenne verbunden ist erlaubt beispielsweise einen Empfang von Daten in dem MICS/MEDS- und MedRadio-Frequenzband. Erfindungsgemäß umfassen die in dem Anschlussgehäuse angeordneten elektrischen Komponenten außerdem einen Schwingkreis der eine Spule und eine Kapazität aufweist und der induktiv mit der Antenne gekoppelt ist. Der Schwingkreis ist auf magnetische Wechselfelder in einem Frequenzbereich zwischen 5 kHz und 50 MHz abgestimmt und hat entsprechend vorzugsweise eine Resonanzfrequenz zwischen 5 kHz und 50 MHz. Der Schwingkreis ist damit für eine Datenkommunikation mittels magnetischer Wechselfelder im Nahfeld geeignet.

Der Schwingkreis für die Datenkommunikation mittels magnetischer Wechselfelder ist somit vorzugsweise von den in dem Hohlgehäuse angeordneten elektrischen Komponenten vollständig galvanisch getrennt.

Die Erfindung erlaubt es, die Spule zur Datenübertragung außerhalb des Hohlgehäuses (nämlich im Anschlussgehäuse des Implantats) anzuordnen. Dadurch kommt die Schirmwirkung des Hohlgehäuses nicht zum Tragen und außerdem ist der störende Einfluss durch die Implantats-Elektronik vernachlässigbar. Als Empfänger dient jetzt nicht mehr eine einzelne Spule sondern vielmehr ein induktiv gekoppeltes System aus einer als Antenne dienenden und mit dem Empfänger oder Transceiver elektrisch verbundenen Spule und einem Schwingkreis, wobei der Schwingkreis eine möglichst hohe Güte aufweist. Der kombinierte Aufbau kann in das Anschlussgehäuse integriert werden.

Im Unterschied zu bekannten implantierbaren medizinischen Geräten braucht der Schwingkreis nicht unmittelbar mit einem Empfänger für die Datenkommunikation mittels magnetischem Wechselfeld verbunden zu sein. Vielmehr haben die Erfinder erkannt, dass der Schwingkreis mit der ebenfalls im Anschlussgehäuse angeordneten Antenne induktiv gekoppelt sein kann sodass den Schwingkreis erregende magnetische Wechselfelder in die für einen wesentlich höheren Frequenzbereich ausgelegte Antenne des implantierbaren medizinischen Gerätes eingekoppelt werden können und so über diese Antenne dem Empfänger des implantierbaren medizinischen Gerätes zugeführt werden können.

Ein Vorteil dieser Lösung ist, dass zwischen Anschlussgehäuse und Hohlgehäuse zum Anschluss der Antenne an den Empfänger beispielsweise nur eine Durchführung vorgesehen zu werden braucht, da der Schwingkreis nicht unmittelbar galvanisch mit dem Empfänger (oder einem zweiten Empfänger) im Hohlgehäuse verbunden zu werden braucht.

Gleichzeitig werden auch die Nachteile vermieden, die sich ergeben, wenn der Schwingkreis für die Datenkommunikation mittels magnetischem Wechselfeld in dem Hohlgehäuse untergebracht wird. Da das Hohlgehäuse typischerweise ein Metallgehäuse ist, werden in das Metallgehäuse eindringende magnetische Wechselfelder durch dieses Metallgehäuse stark abgeschwächt (wenn auch nicht vollständig abgeschirmt). Dies ist bei einem typischerweise aus Kunststoff, z.B. Epoxidharz, oder einem anderen nicht leitendem Material bestehenden Anschlussgehäuse des implantierbaren medizinischen Gerätes nicht der Fall. Die Spule zur Datenübertragung ist bei den heute verfügbaren Implantaten im Titan-Gehäuse angeordnet. Es handelt sich um ein einfaches System aus zwei Spulen. Bei herkömmlichen implantierbaren medizinischen Geräten mit in ein Titan-Gehäuse integrierter Spule wird das Magnetfeld im Inneren des Hohlgehäuses durch das Hohlgehäuse geschwächt, wodurch die Reichweite verringert wird. Außerdem kommt es bei herkömmlichen implantierbaren medizinischen Geräten zu Störeinstrahlung in die Kommunikationsspule durch die restliche Elektronik des Implantats.

Soll bei herkömmlichen implantierbaren medizinischen Geräten zudem ein Schwingkreis zur Optimierung der Reichweite Verwendung finden, ist seine Güte sehr niedrig. Dies hat seine Ursache in den Wirbelstromverlusten, die insbesondere aus der Nähe zum Metall des Hohlgehäuses in der Umgebung der Spule resultieren. Durch das erfindungsgemäße implantierbare medizinische Gerät werden diese Nachteile vermieden.

Ein im Anschlussgehäuse (und nicht im Hohlgehäuse) angeordneter Schwingkreis für die Datenkommunikation mittels magnetischem Wechselfeld bietet darüber hinaus den Vorteil, dass die Datenkommunikation bei höheren Frequenzen beispielsweise bis in den Bereich von 50 MHz erfolgen kann. Dies erlaubt kleinere Bauteile, insbesondere kleine Spulen. Derartige Frequenzen können nicht verwendet werden, wenn die Spule für die Datenkommunikation mittels magnetischem Wechselfeld in einem metallischen Hohlgehäuse angeordnet ist, weil Frequenzen im Bereich über 50 MHz durch das Metallgehäuse zu stark gedämpft werden und höhere Frequenzen praktisch ganz abgeschirmt werden.

Mit Hilfe der vorliegenden Erfindung lässt sich die Reichweite bei der Datenübertragung mit Hilfe von zwei induktiv miteinander gekoppelten Spulen vergrößern.

Klassisch kommen beim Aufwecken von Implantaten Magnetsensoren zum Einsatz. Hier kommt es allerdings bei Patienten mit hohem Body-Mass-Index (BMI) bereits zu Kommunikationsproblemen. Durch die vergrößerte Reichweite des erfindungsgemäßen Verfahrens wird es auch sinnvoll möglich, die induktive Kopplung zum Aufwecken von Implantaten zu benutzen. Bei der erfindungsgemäßen induktiven Kopplung kann das Aufwecken dadurch erfolgen, dass das Signal passiv gleichgerichtet wird und anschließend einem Schwellwertentscheider zugeführt wird. Das induktiv übertragene Signal erzeugt in den induktiv miteinander gekoppelten Spulen einen Wechselstrom, der gleichgerichtet und mit einem Schwellwert verglichen werden kann, so dass bei ausreichend großer Amplitude des gleichgerichteten Signals ein Einschaltsignal generiert wird. Das implantierbare medizinische Gerät kann hierzu einen Gleichrichter und einen Komparator enthalten, die elektrisch mit den induktiv miteinander gekoppelten Spulen verbunden sind.

Wenn zusätzlich gemäß einer bevorzugten Ausführungsvariante der Erfindung der Abschlusswiderstand der Antenne an den ohmschen Widerstand des Schwingkreises leistungsangepasst ist, wird auch die durch den Gütefaktor Q auszudrückende Güte des Schwingkreises nicht durch die Antenne beeinträchtigt.

Um eine derartige Leistungsanpassung zu erzielen, ist der Abschlusswiderstand $R_L$ vorzugsweise so dimensioniert, dass er folgender Gleichung genügt:

$$R_L = R_1 \left( \frac{N_2}{N_1} \right)^2 ,$$

wobei $N_1$ die Anzahl der Windungen ist, die die Spule des Schwingkreises hat und $N_2$ die Anzahl der Windungen ist, die die Antenne hat und $R_1$ der ohmsche Widerstand ist, der in dem Schwingkreis wirkt.

Die Antenne wird vorzugsweise von der Spule gebildet und zwar insbesondere von einer Spule mit einer einzigen Windung. Dementsprechend ist $N_2=1$.

Die Spule des Schwingkreises ist vorzugsweise eine Spule mit mehreren Windungen $N_1$.

Vorzugsweise ist die Spule des Schwingkreises wenigstens annähernd konzentrisch und/oder parallel zu der die Antenne bildenden Spule ausgerichtet, um so eine optimale magnetische Kopplung der beiden Spulen zu erzielen.

Die Kapazität des Schwingkreises ist vorzugsweise von einem Kondensator gebildet. Alternativ kann die Kapazität aber auch von einer Eigenkapazität der Spule selbst gebildet sein.

Vorzugsweise werden für die Anregung des Schwingkreises - also für die Datenkommunikation mittels magnetischem Wechselfeld - Impulse von mehr als 10$\mu$s Dauer verwendet, um sicherzustellen, dass der Schwingkreis mit seiner hohen Güte genügend Zeit zum Anschwingen hat.

Die für die Datenkommunikation mittels magnetischem Wechselfeld genutzten Frequenzen sind beispielsweise 125 kHz, 13,56 MHz und 37,5 MHz. Bei größerer Frequenz (z.B. ab 2 MHz bis 37,5 MHz) vereinfacht sich die Spule des Schwingkreises auf wenige Windungen. Bei den höheren der genannten Frequenzen wäre eine Kommunikation durch ein metallisches Hohlgehäuse hindurch ausgeschlossen.

Für eine Datenkommunikation bei höheren Frequenzen zwischen beispielsweise 2 MHz und 40 MHz kann der Schwingkreis von einer Spule mit Eigenresonanz gebildet sein.

Als Material für den Schwingkreis und insbesondere dessen Spule ist vorzugsweise ein gut leitfähiges Metall erforderlich, um die bestmögliche Güte zu erreichen. Je nach Anzahl der Windungen der Spule ist allerdings der Einfluss der Leitfähigkeit des Metalls vernachlässigbar. Als Material für die Spule besonders geeignet ist ein Draht aus Silber, gefolgt von Gold, gefolgt von Titan, gefolgt von Edelstahl und gefolgt von Nitinol. Gemäß einer vorteilhaften Ausführungsvariante wird der Schwingkreis von einer eigenresonanten Spule gebildet. Diese kann eine Helix-Spule sein. Geeignet ist beispielsweise eine Spule mit einer Selbstresonanz bei 39,7MHz. Diese kann bei dieser Frequenz eine Kreisgüte von Q=277 haben. Beispielsweise hat die Spule 65 Windungen aus einem 50 $\mu$m dicken Draht, der mit geringem Abstand gewickelt ist, sodass die Spule einen Durchmesser von 10mm hat. Die Spule hat dann eine Gesamtlänge von etwa 4mm. Durch geringfügiges optimieren lässt sich die Eigenresonanz anpassen, z. B. auf 37,5 MHz. Eine Frequenz von 37,5 MHz wird beispielsweise von REG70.03 für Implantate mit extrem niedrigem Energiebedarf genannt.

Das Hohlgehäuse ist vorzugsweise ein Metallgehäuse aus einem biokompatiblen Metall wie beispielsweise rostfreiem Stahl oder Titan. Ein Vorteil eines derartigen Metallgehäuses ist es, dass die elektrischen Komponenten im Inneren des Hohlgehäuses vor von außen wirkender elektromagnetischer Strahlung abgeschirmt sind. Daher ist es auch vorteilhaft, wenn das Anschlussgehäuse im Wesentlichen von einem nicht leitenden Material wie beispielsweise Kunststoff gebildet ist, da dann die in dem Anschlussgehäuse angeordneten Komponenten wie Antenne und Schwingkreis gerade nicht gegenüber elektromagnetischen beziehungsweise magnetischen Wechselfeldern abgeschirmt sind.

Außerdem weist das Anschlussgehäuse vorzugsweise Anschlüsse zum Anschluss von elektrischen Leitungen beispielsweise Stimulations- oder Sensingelektrodenleitungen auf.

In dem Hohlgehäuse sind vorzugsweise weitere elektrische Komponenten wie insbesondere eine Batterie angeordnet.

Der in dem Hohlgehäuse angeordnete Empfänger ist vorzugsweise als Sender und Empfänger ausgebildet, also als Transceiver, der Signale im 3 bis 4-stelligen Megahertzbereich empfangen und senden kann und der darüber hinaus Signale im Kilohertzbereich bis 2-stelligem Megahertzbereich empfangen kann.

Erfindungsgemäß wird auch ein Verfahren zur drahtlosen Datenkommunikation zwischen einem externen Gerät und einem implantierbaren medizinischen Gerät mittels magnetischer Wechselfelder vorgeschlagen, bei dem Daten induktiv von dem externen Gerät zu einem Schwingkreis des implantierbaren medizinischen Gerätes übertragen werden, der seinerseits mit einer Antennenspule induktiv gekoppelt ist, sodass von dem Schwingkreis empfangene Signale induktiv in die Antennenspule eingekoppelt werden und über diese an einem mit der Antennenspule elektrisch verbundenen Empfänger oder Sender/Empfänger weitergeführt werden.

Vorzugsweise erfolgt die Datenkommunikation mittels magnetischem Wechselfeld bei einer Frequenz zwischen 2 MHz und 50 MHz.

Vorzugsweise wird das Verfahren mit einem implantierbaren medizinischen Gerät der hier beschriebenen Art durchgeführt.

Die Erfindung soll nun anhand von Ausführungsbeispielen in Bezug auf die Figuren näher erläutert werden.

Von den Figuren zeigt

Fig. 1: ein erfindungsgemäßes implantierbares medizinisches Gerät;

Fig. 2: ein implantierbares medizinisches Gerät gemäß dem Stand der Technik;

Fig. 3: einige elektrische Komponenten eines erfindungsgemäßen implantierbaren medizinischen Gerätes;

Fig. 4: eine Prinzipdarstellung einer Datenkommunikation mit dem erfindungsgemäßen implantierbaren medizinischen Gerät im Nahfeld über ein magnetisches Wechselfeld;

Fig. 5: eine schematische Darstellung der Kopplung eines Schwingkreises und einer Antenne des erfindungsgemäßen implantierbaren medizinischen Gerätes über ein magnetisches Wechselfeld;

Fig. 6: eine schematische Darstellung der Kopplung eines externen Gerätes wie beispielsweise eines Programmiergerätes mit dem erfindungsgemäßen implantierbaren medizinischen Gerät im Nahfeld über ein magnetisches Wechselfeld;

Fig. 7: ein Beispiel für eine selbstresonante Helixspule, die als Schwingkreis für das erfindungsgemäße implantierbare medizinische Gerät benutzt werden kann; und

Fig. 8: eine Darstellung des Impedanzverlaufs der in Figur 7 dargestellten Spule.

Das in Figur 1 dargestellte implantierbare medizinische Gerät 10 besitzt ein Hohlgehäuse 12, das mit einem Anschlussgehäuse 14 verbunden ist. An das Anschlussgehäuse 14 ist eine Stimulationselektrodenleitung 16 angeschlossen.

In dem Anschlussgehäuse 14 sind eine Antenne 20 und ein Schwingkreis 22 angeordnet. Die Antenne 20 ist galvanisch mit einem Empfänger 24 verbunden, der im Inneren des Hohlgehäuses 12 angeordnet ist. Der Schwingkreis 22 ist über ein magnetisches Wechselfeld mit der Antenne 20 gekoppelt.

Der Schwingkreis 22 weist eine Spule 26 sowie einen Kondensator 28 auf, der eine Kapazität des Schwingkreises 22 bildet.

Figur 2 zeigt ein implantierbares medizinisches Gerät gemäß dem Stand der Technik, das einen Sender/Empfänger (Transceiver) TX in einem Hohlgehäuse aufweist, der an eine Antenne angeschlossen ist, die in dem Anschlussgehäuse des implantierbaren medizinischen Gerätes angeordnet ist. Wie sich aus dem Vergleich der Figuren 1 und 2 ergibt, weist das erfindungsgemäße implantierbare medizinische Gerät 10 zusätzlich den in dem Anschlussgehäuse 14 angeordneten Schwingkreis 22 für die induktive Datenübertragung auf.

Figur 3 zeigt einige der elektronischen Komponenten des implantierbaren medizinischen Gerätes 10 in detaillierterer Darstellung. Wie Figur 3 zu entnehmen ist, ist der Sender/Empfänger 24 über eine Durchführung 30 mit der Antenne 20 verbunden, die von einer Spule mit einer einzigen Windung gebildet ist.

Außerdem zeigt Figur 3 den Schwingkreis 22, der eine Spule 26 mit mehreren Windung $N_1$ sowie eine von einem Kondensator 28 gebildete Kapazität besitzt. Wie Figur 3 zeigt, können die die Antenne 20 bildende Spule sowie die Spule 26 des Schwingkreises 22 exakt übereinanderliegend angeordnet sein.

Figur 4 dient der Erläuterung einer induktiven Datenübertragung von einem Patientengerät mit einer Induktionsspule 40 zum implantierbaren medizinischen Gerät mit seinem Schwingkreis 22, das die Spule 26 und die Kapazität 28 aufweist. Wie Figur 4 zu entnehmen ist, ist die induktive Kopplung zwischen der Spule 40 des externen Geräts und der Spule 26 des Schwingkreises 22 schwach.

Innerhalb des Anschlussgehäuses 14 besteht auf der anderen Seite eine starke induktive Kopplung zwischen der Spule 26 des Schwingkreises 22 und der die Antenne 20 bildenden Spule.

Figur 4 deutet ebenfalls an, dass die die Antenne bildende Spule mit einem in dem Hohlgehäuse (also im Implantat) angeordneten Abschlusswiderstand $R_L$ abgeschlossen ist.

Figur 5 verdeutlicht die starke induktive Kopplung zwischen der als Spule ausgebildeten Antenne 20 und der Spule 26 des Schwingkreises 22. Angedeutet sind die magnetischen Feldlinien 42 des Magnetfeldes, das zwischen der Antenne 20 und der Spule 26 des Schwingkreises 22 wirkt.

Figur 6 illustriert die vergleichsweise schwächere induktive Kopplung zwischen einer Spule 40 eines externen Gerätes und der Spule 26 des Schwingkreises 22 im Anschlussgehäuse 14 des implantierbaren medizinischen Gerätes 10. Auch in Figur 6 sind wieder Magnetfeldlinien 44 angedeutet, die das Magnetfeld repräsentieren, über das die Spule 40 des externen Gerätes induktiv mit der Spule 26 des Schwingkreises 22 gekoppelt ist.

Die in Figuren 1, 3 und 4 vorgestellte Lösung basiert auf der Verwendung der MICS-Antenne 20, die bereits im Anschlussgehäuse 14 des aktiven Implantats 10 vorhanden ist. Diese Antenne 20 wird über eine Durchführung 30 auf die Innenseite des Hohlgehäuses 12 geführt. Dort ist die Antenne über ein Anpassnetzwerk mit dem MICS-Transceiver 24 verbunden.

Die MICS-Antenne 20 wird von einer Spule mit nur einer Windung gebildet, siehe Figuren 1 und 3. Zusätzlich zu dieser Spule der Antenne 20 ist im Anschlussgehäuse 14 ein Schwingkreis 22 angeordnet, der seine Resonanzfrequenz bei der Trägerfrequenz der induktiven Datenübertragung hat und eine weitere Spule 26 und einen Kondensator 28 aufweist; siehe Figur 1. Dieser Schwingkreis 22 ist induktiv mit der MICS-Antenne 20 gekoppelt; siehe Figuren 4 und 5.

Durch optimale Konfiguration von MICS-Antenne 20 und Schwingkreis 22 - insbesondere durch Variieren von Kopplungsfaktor und Windungszahl - lässt sich eine Anpassung zwischen dem Schwingkreis 22 und dem Abschlusswiderstand $R_L$ der MICS-Antenne 20 erreichen. Im Gegensatz zu konventionellen Methoden wird bei diesem Vorgehen die Güte des Schwingkreises 22 nicht durch das Einfügen in einen elektrischen Schaltkreis verringert, wodurch sich eine größere Reichweite erzielen lässt. Figur 6 zeigt die induktive Kopplung zwischen einem Patientengerät außerhalb des Körpers und einem aktiven Implantat mit den beschriebenen gekoppelten Spulen.

Um die gewünschte Leistungsanpassung zu erzielen und die Güte Q des Schwingkreises 22 nicht zu beeinträchtigen, ist die Antenne 20 vorzugsweise mit einem ohmschen Abschlusswiderstand $R_L$ abgeschlossen, der folgender Gleichung genügt:

$$R_1 = R_L\left(\frac{N_1}{N_2}\right)^2,$$

$R_1$ ist dabei der ohmsche Widerstand, der in dem Schwingkreis wirkt. $N_1$ ist die Anzahl der Windungen der Spule des Schwingkreises und $N_2$ ist die Anzahl der Windungen der die Antenne bildenden Spule.

Eine Möglichkeit zur konkreten Umsetzung ist bereits in Figuren 1 und 3 gezeigt. Hier liegen beide Spulen exakt übereinander. In einer anderen Ausführungsform könnte der Schwingkreis 22 beispielsweise eine deutlich kleinere Fläche einnehmen als die MICS-Antenne 20. Die Resonanzfrequenz hängt von der durch Größe (Geometrie) und Windungszahl der Spule bestimmten Induktivität der Spule und den elektrischen Eigenschaften, insbesondere der intrinsischen Kapazität und den übrigen, mit der Spule gekoppelten elektrischen Komponenten ab.

Gemäß einer alternativen Ausführungsform kann eine Eigenresonanzfrequenz der Spule 26' des Schwingkreises 20 für die Datenübertragung genutzt werden; siehe Figuren 7 und 8. Dadurch kann auf zusätzliche Bauteile im Anschlussgehäuse 14 verzichtet werden. Ein Beispiel für eine solche eigenresonante Spule 26' ist in Figur 7 gezeigt. Figur 8 zeigt den zugehörigen Impedanzverlauf der in Figur 7 dargestellten eigenresonanten Spule 26'. Wie diesem Impedanzverlauf zu entnehmen ist, hat die Spule 26' eine Selbstresonanz bei 39,7 MHz. Wenn für den Schwingkreis 22 eine solche eigenresonante Spule 26' verwendet wird, braucht der Schwingkreis 22 keinen zusätzlichen Kondensator, vielmehr reicht die Kapazität der eigenresonanten Spule 26' als (einzige) Kapazität des Schwingkreises 22.

Wie sich aus Figur 7 entnehmen lässt, hat eine geeignete eigenresonante Spule 26' einen Durchmesser von 10 mm und eine Länge von etwa 3,9 mm und weist 65 Windungen auf ($N_1$=65). Die Spule ist als Helix gewickelt und hat eine Gesamtlänge von etwa 4 mm. Die eigenresonante Spule 26' ist von einem Draht aus gut leitfähigem Metall mit einem Drahtdurchmesser von 50 $\mu$m gebildet. Die Steigung der Spulenwindungen beträgt 60 $\mu$m, d. h. die nebeneinander liegenden Drähte der einzelnen Spulenwindungen haben einen Abstand von etwa 10 $\mu$m voneinander. Figur 7A zeigt schematisch eine Seitenansicht einer derartigen geeigneten eigenresonanten Spule 26' und Figur 7B die zugehörige Draufsicht. Wie der Figur 7B zu entnehmen ist, ist die eigenresonante Spule 26' in der Draufsicht kreisförmig.

Als Material für die Spule 26' besonders geeignet ist ein gut leitfähiges Metall, beispielsweise ein Draht aus Silber, gefolgt von Gold, gefolgt von Titan, gefolgt von Edelstahl und gefolgt von Nitinol.

Die Spule 26' hat eine Selbstresonanz bei 39,7 MHz. und hat bei dieser Frequenz eine Kreisgüte von Q=277. Durch geringfügiges optimieren lässt sich die Eigenresonanz auf 37,5 MHz ändern.

Die vorgestellte induktive Telemetrie mit großer Reichweite und gleichzeitig verringerter Implantatsinterner Störeinstrahlung ermöglicht durch die vergrößerte Reichweite auch ein energiesparendes Aktivieren des Implantats. Hierzu kann das implantierbare medizinische Gerät einen Gleichrichter und einen Komparator enthalten, die elektrisch mit den induktiv miteinander gekoppelten Spulen verbunden sind. Das induktiv übertragene Signal erzeugt in den induktiv miteinander gekoppelten Spulen einen Wechselstrom, der gleichgerichtet und mit einem Schwellwert verglichen werden kann, so dass bei ausreichend großer Amplitude des gleichgerichteten Signals ein Einschaltsignal generiert werden kann.

**Bezugszeichenliste**

| | |
|---|---|
| 10 | Implantierbares medizinisches Gerät |
| 12 | Hohlgehäuse |
| 14 | Anschlussgehäuse |
| 16 | Stimulationselektrodenleitung |
| 20 | Antenne |
| 22 | Schwingkreis |
| 24 | Sender/Empfänger (Transceiver) |
| 26, 26 | Spule des Schwingkreises 22 |
| 28 | Kondensator des Schwingkreises 22 |
| 30 | Durchführung |
| 40 | Spule eines externen Gerätes |
| 42,44 | Magnetfeldlinien |

**Patentansprüche**

1. Implantierbares medizinisches Gerät (10) mit einem Hohlgehäuse (12) und einem Anschlussgehäuse (14), das mit dem Hohlgehäuse (12) verbunden ist, wobei sowohl das Hohlgehäuse (12) als auch das Anschlussgehäuse (14) elektrische Komponenten enthalten, von denen die in dem Hohlgehäuse (12) enthaltenen elektrischen Komponenten einen Empfänger (24) für drahtlose Kommunikation umfassen und die im Anschlussgehäuse (14) enthaltenen Komponenten eine Antenne (20), die zum Empfang elektromagnetischer Wellen mit einer Frequenz von mehr als 100 MHz konfiguriert ist und die elektrisch mit dem Empfänger (24) verbunden ist, **dadurch gekennzeichnet, dass** die in dem Anschlussgehäuse (14) enthaltenen elektrischen Komponenten außerdem einen Schwingkreis (22) umfassen, der eine Spule (26) und eine Kapazität (28) aufweist und der induktiv mit der Antenne (20) gekoppelt ist, wobei der Schwingkreis (22) konfiguriert ist auf magnetische Wechselfelder in einem Frequenzbereich zwischen 5 kHz und 50 MHz anzusprechen.

2. Implantierbares medizinisches Gerät (10) nach Anspruch 1, **dadurch gekennzeichnet, dass** der Empfänger (24) in dem Hohlgehäuse (12) und die Antenne (20) in dem Anschlussgehäuse (14) über eine Durchführung, (30) elektrisch miteinander verbunden sind.

3. Implantierbares medizinisches Gerät (10) nach Anspruch 1 oder 2, **dadurch gekennzeichnet, dass** die Antenne (20) von einer Spule mit einer einzigen Windung gebildet ist.

**4.** Implantierbares medizinisches Gerät (10) nach wenigstens einem der Ansprüche 1 bis 3, **dadurch gekennzeichnet, dass** die Spule (26; 26') des Schwingkreises (22) eine Spule mit mehreren Windungen ist.

**5.** Implantierbares medizinisches Gerät (10) nach Anspruch 3 und 4, **dadurch gekennzeichnet, dass** die Spule (26; 26') des Schwingkreises (22) wenigstens annähernd konzentrisch und/oder parallel zu der die Antenne (20) bildenden Spule ausgerichtet ist.

**6.** Implantierbares medizinisches Gerät (10) nach wenigstens einem der Ansprüche 1 bis 5, **dadurch gekennzeichnet, dass** die Antenne (20) und der Schwingkreis (22) derart aneinander leistungsangepasst sind, dass die Güte des Schwingkreises (22) nicht beeinträchtigt ist.

**7.** Implantierbares medizinisches Gerät (10) nach Anspruch 6, **dadurch gekennzeichnet, dass** die Antenne (20) mit einem Abschlusswiderstand ($R_L$) abgeschlossen ist, der wie folgt dimensioniert ist:

$$R_L = R_1 \left( \frac{N_2}{N_1} \right)^2 ,$$

wobei $N_1$ die Anzahl der Windungen ist, die die Spule des Schwingkreises hat und $N_2$ die Anzahl der Windungen ist, die die Antenne hat und $R_1$ der ohmsche Widerstand ist, der in dem Schwingkreis wirkt.

**8.** Implantierbares medizinisches Gerät (10) nach wenigstens einem der Ansprüche 1 bis 7, **dadurch gekennzeichnet, dass** der Schwingkreis (22) eine Resonanzfrequenz zwischen 5 kHz und 50 MHz hat.

**9.** Implantierbares medizinisches Gerät (10) nach wenigstens einem der Ansprüche 1 bis 8, **dadurch gekennzeichnet, dass** die Kapazität des Schwingkreises (22) von einem Kondensator (28) gebildet ist.

**10.** Implantierbares medizinisches Gerät (10) nach wenigstens einem der Ansprüche 1 bis 8, **dadurch gekennzeichnet, dass** der Schwingkreis (22) von einer eigenresonanten Spule (26') mit einer Eigenresonanzfrequenz zwischen 2 MHz und 50 MHz gebildet ist.

**11.** Implantierbares medizinisches Gerät (10) nach wenigstens einem der Ansprüche 1 bis 10, **dadurch gekennzeichnet, dass** der Schwingkreis (22) eine Güte Q hat, die größer 200 ist.

**12.** Verfahren zur drahtlosen Datenkommunikation zwischen einem externen Gerät und einem implantierbaren medizinischen Gerät (10) mittels magnetischer Wechselfelder, bei dem Daten induktiv von dem externen Gerät zu einem Schwingkreis des implantierbaren medizinischen Gerät übertragen werden, der seinerseits mit einer Antennenspule induktiv gekoppelt ist, sodass von dem Schwingkreis empfangene Signale induktiv in die Antennenspule eingekoppelt werden und über diese an einen mit der Antennenspule elektrisch verbundenen Empfänger oder Sender/Empfänger weitergeführt werden.

**13.** Verfahren nach Anspruch 12, **dadurch gekennzeichnet, dass** die Datenkommunikation mittels magnetischem Wechselfeld bei einer Frequenz zwischen 2 MHz und 50 MHz durchgeführt wird.

**14.** Verfahren nach Anspruch 12, **dadurch gekennzeichnet, dass** das Verfahren mit einem implantierbaren medizinischen Gerät (10) nach wenigstens einem der Ansprüche 1 bis 11 durchgeführt wird.

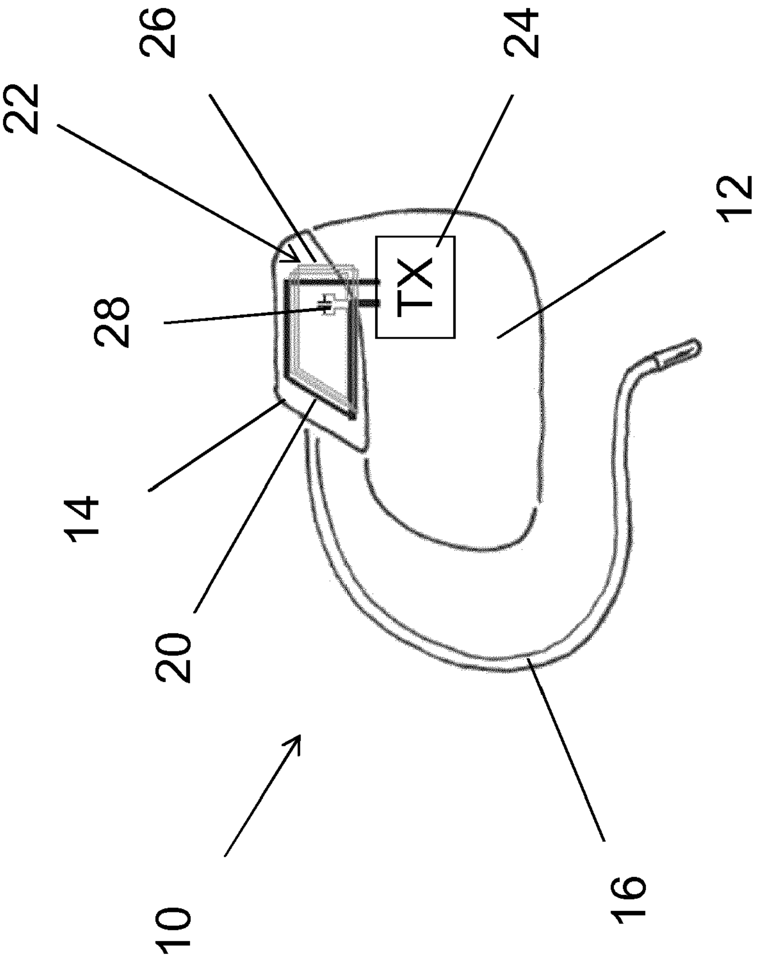


Fig. 1

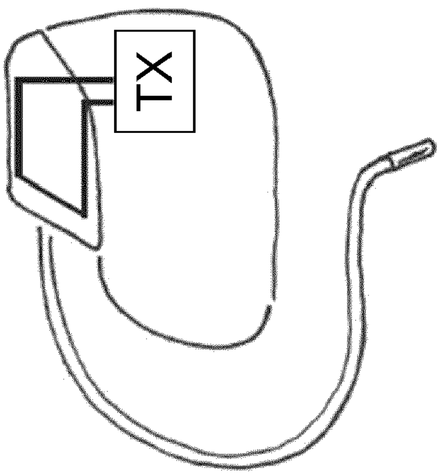


Fig. 2

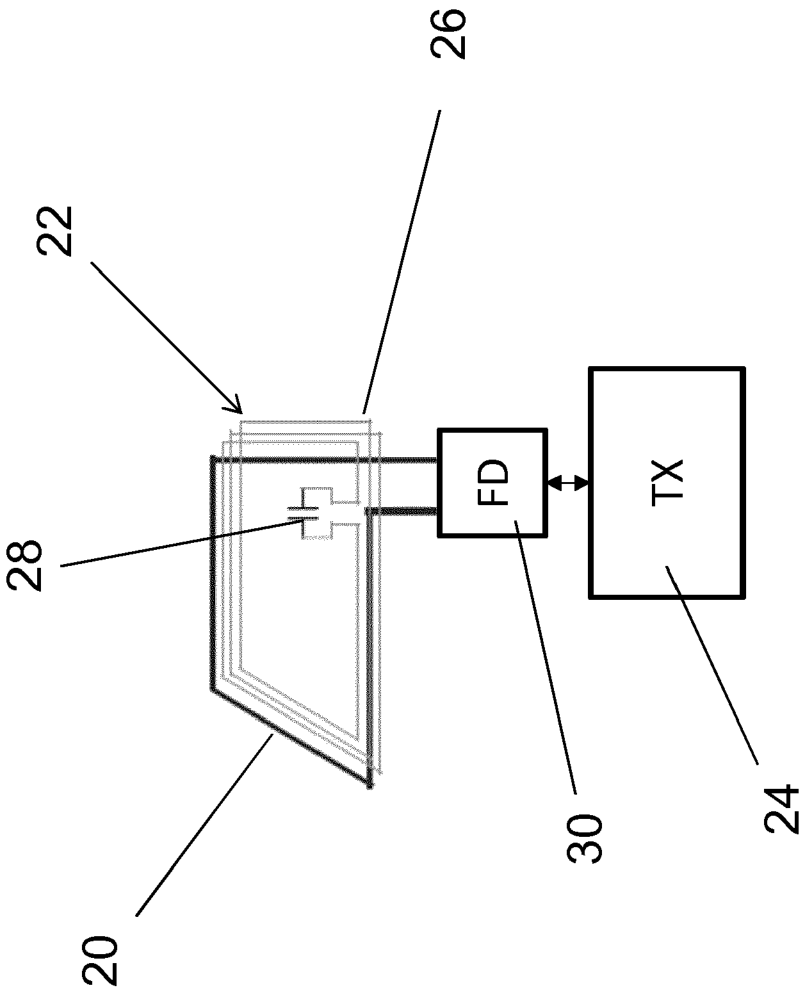


Fig. 3

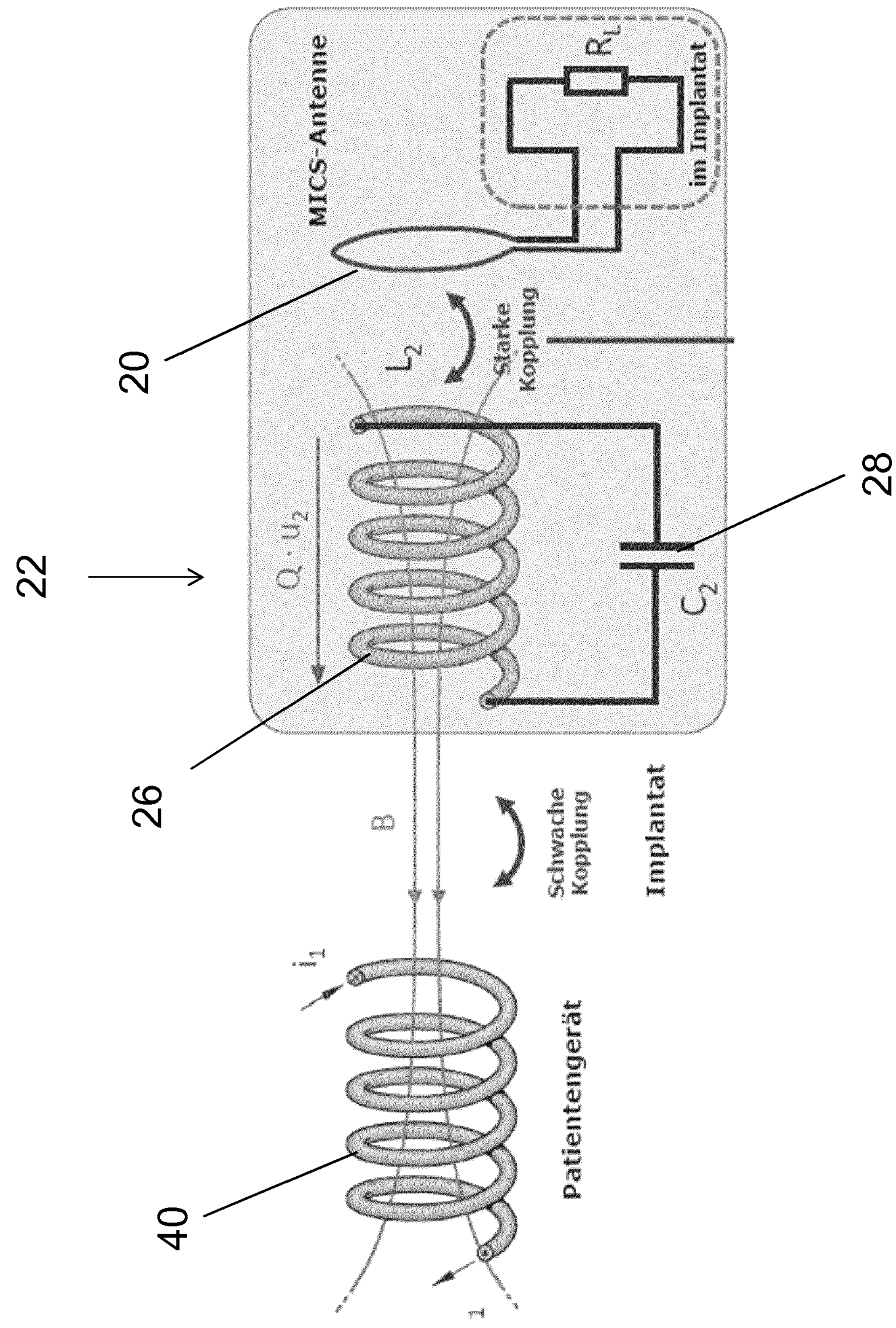
20
22
26
28
40
MICS-Antenne
$R_L$
im Implantat
$L_2$
Starke Kopplung
$Q \cdot u_2$
$C_2$
B
Schwache Kopplung
Implantat
$i_1$
Patientengerät

Fig. 4

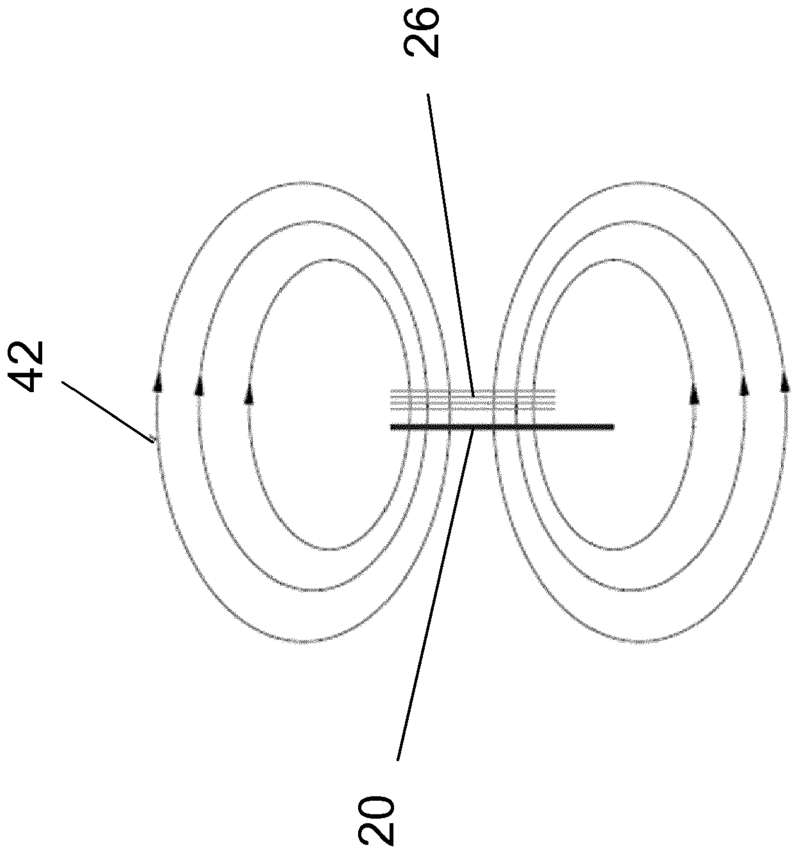


Fig. 5

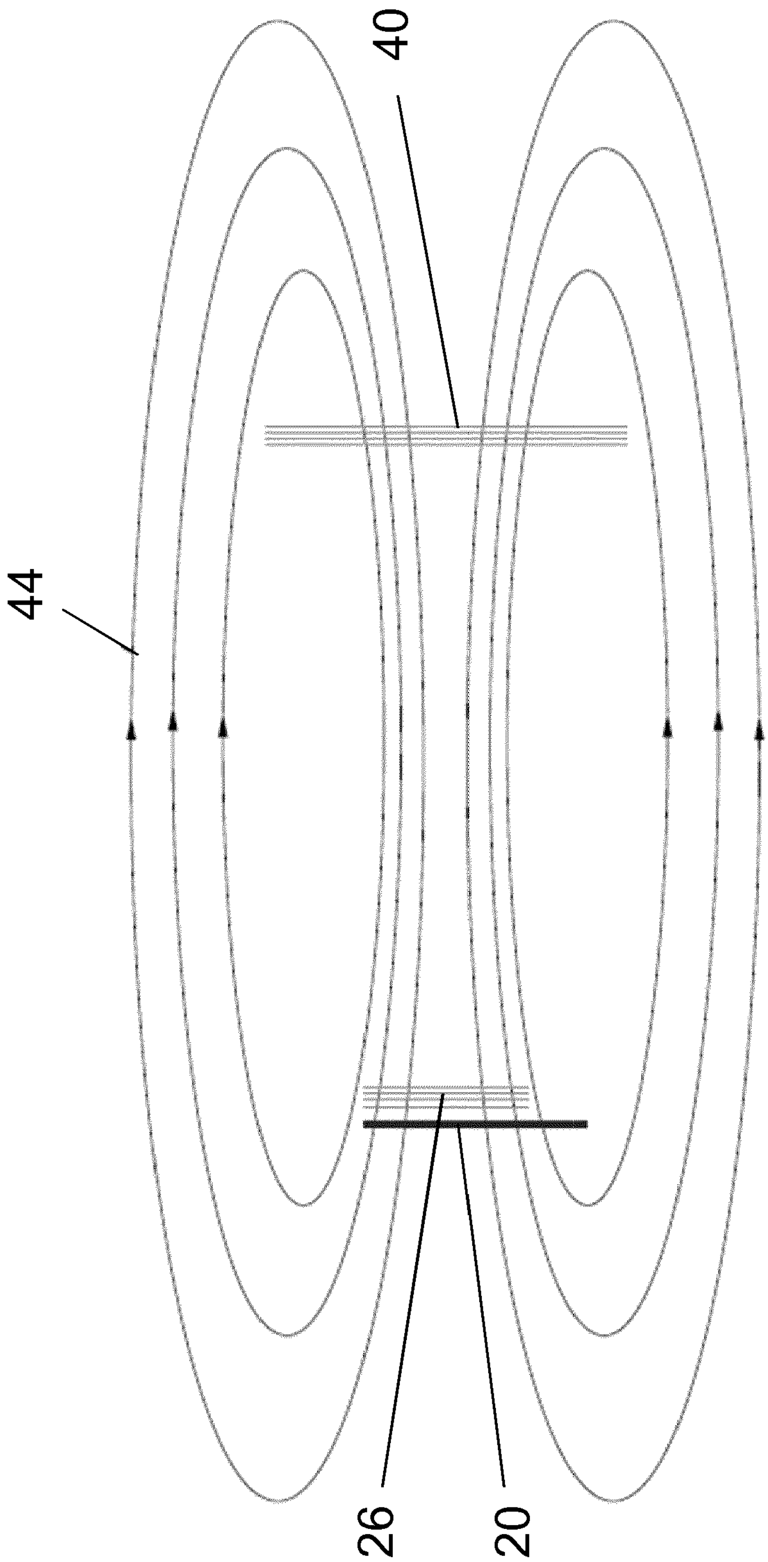


Fig. 6

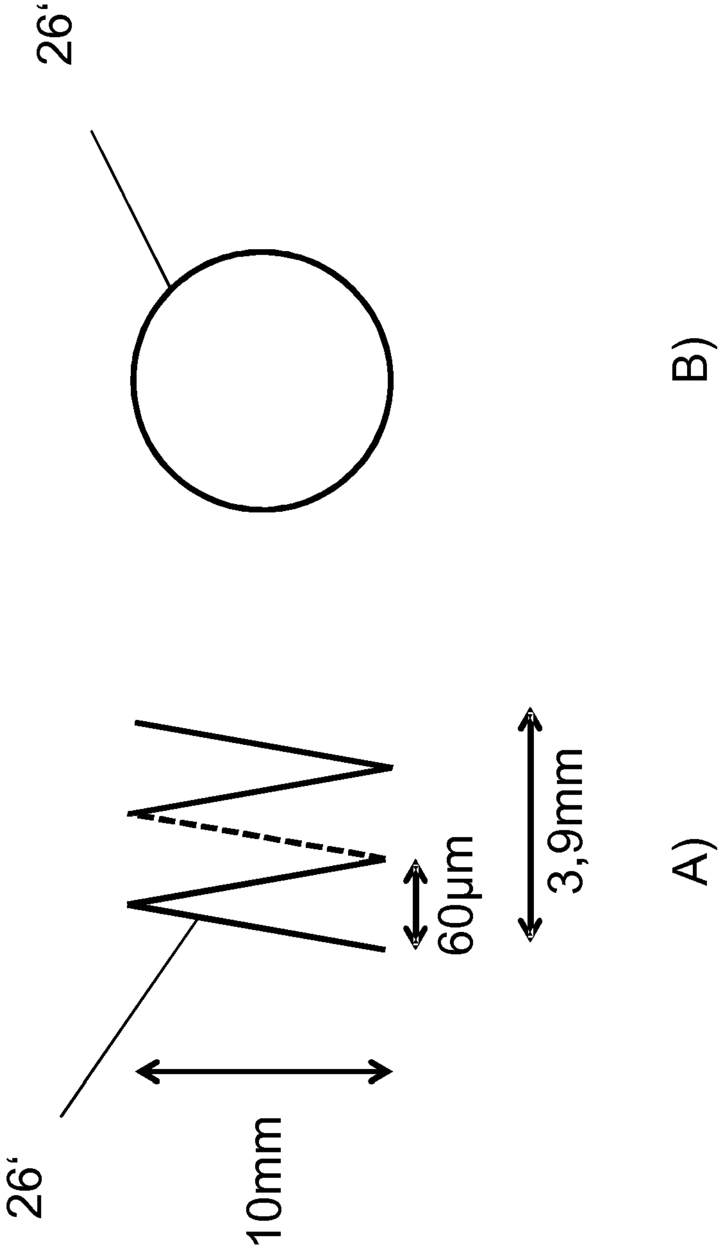


Fig. 7

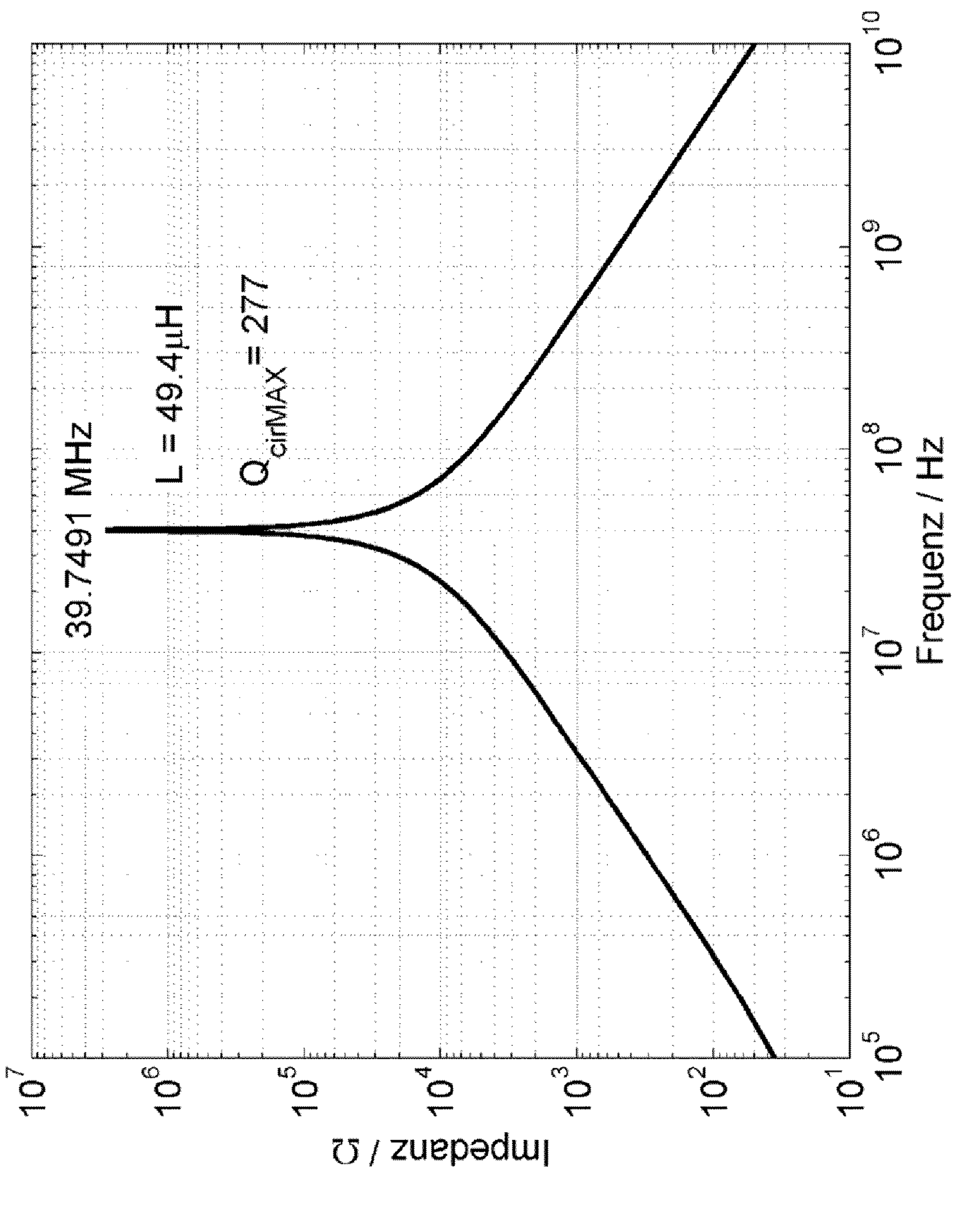
39.7491 MHz
L = 49.4μH
Q_cirMAX = 277
Impedanz / Ω
Frequenz / Hz
10^1
10^2
10^3
10^4
10^5
10^6
10^7
10^5
10^6
10^7
10^8
10^9
10^10

Fig. 8

Europäisches Patentamt
European Patent Office
Office européen des brevets

# EUROPÄISCHER RECHERCHENBERICHT

Nummer der Anmeldung
EP 16 17 9171

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | EP 2 111 895 A2 (BIOTRONIK CRM PATENT AG [CH]) 28. Oktober 2009 (2009-10-28)<br>* Zusammenfassung; Abbildung 2 *<br>* Absatz [0023] *<br>----- | 1-14 | INV.<br>A61N1/375<br>A61N1/372 |
| A | US 7 650 191 B1 (LIM WISIT [US] ET AL) 19. Januar 2010 (2010-01-19)<br>* Zusammenfassung; Abbildung 9 *<br>----- | 1-14 | |
| A | US 2003/018369 A1 (THOMPSON DAVID L [US] ET AL) 23. Januar 2003 (2003-01-23)<br>* Zusammenfassung; Abbildungen 3, 4 *<br>----- | 1-14 | |

RECHERCHIERTE SACHGEBIETE (IPC)

A61N

Der vorliegende Recherchenbericht wurde für alle Patentansprüche erstellt

1

| Recherchenort | Abschlußdatum der Recherche | Prüfer |
|---|---|---|
| München | 16. Januar 2017 | Wetzig, Thomas |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 16 17 9171

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

16-01-2017

| Im Recherchenbericht angeführtes Patentdokument | Datum der Veröffentlichung | Mitglied(er) der Patentfamilie | Datum der Veröffentlichung |
|---|---|---|---|
| EP 2111895 A2 | 28-10-2009 | DE 102008021064 A1 | 29-10-2009 |
| | | EP 2111895 A2 | 28-10-2009 |
| | | US 2009270961 A1 | 29-10-2009 |
| US 7650191 B1 | 19-01-2010 | KEINE | |
| US 2003018369 A1 | 23-01-2003 | CA 2454020 A1 | 30-01-2003 |
| | | DE 60223146 T2 | 31-07-2008 |
| | | EP 1409075 A2 | 21-04-2004 |
| | | JP 4299661 B2 | 22-07-2009 |
| | | JP 2004538058 A | 24-12-2004 |
| | | US 2003018369 A1 | 23-01-2003 |
| | | US 2004078067 A1 | 22-04-2004 |
| | | US 2004176822 A1 | 09-09-2004 |
| | | WO 03008037 A2 | 30-01-2003 |

EPO FORM P0461

Für nähere Einzelheiten zu diesem Anhang : siehe Amtsblatt des Europäischen Patentamts, Nr.12/82